# EUROPEAN PATENT SPECIFICATION

(11) **EP 0 755 403 B1**
(45) Date of publication and mention of the grant of the patent: **11.08.1999**
(21) Application number: 95917474.9
(22) Date of filing: 26.04.1995
(51) Int. Cl.: C07K 1/36, C07K 14/235, A61K 39/10

(54) **METHOD OF SEPARATING PROTECTIVE COMPONENTS OF BORDETELLA PERTUSSIS**
VERFAHREN ZUR TRENNUNG VON PROTEKTIVEN VERBINDUNGEN AUS BORDETELLA PERTUSSIS
PROCEDE PERMETTANT DE SEPARER DES ELEMENTS PROTECTEURS DE BORDETELLA PERTUSSIS

(30) Priority: 28.04.1994 JP 9156594
(43) Date of publication of application: 29.01.1997
(73) Proprietor: Takeda Chemical Industries, Ltd., Osaka-shi, Osaka 541-0045 (JP)
(72) Inventor: SUEHARA, Akihiro, Yamaguchi 743 (JP); YAMAMOTO, Eiji, Yamaguchi 743-01 (JP); FUJII, Shigeo, Yamaguchi 743-01 (JP)
(74) Representative: von Kreisler, Alek, Dipl.-Chem.
(86) International application number: PCT/JP95/00830
(87) International publication number: WO 95/29934

(56) References cited:
- EP-A- 0 202 947
- EP-A- 0 231 083
- EP-A- 0 291 968
- EP-A- 0 484 621
- THE JOURNAL OF MEDICAL MICROBIOLOGY, vol. 35, 1991 page 187 VOSE 'A NEW ACELLULAR PERTUSSIS VACCINE'

## Description

### Technical Field

The present invention relates to a method of separating protective components of Bordetella pertussis. The pertussis component vaccine can be produced by suitably mixing the protective components separated by the method of the present invention.

### Background art

Vaccines are widely used to prevent communicable diseases. Pertussis, a communicable respiratory disease caused by infection with Bordetella pertussis, is likely to severely affect patients, especially infants, due to apneic cough with occasional spasm. To cope with this disease, it has been common practice to use whole cultured cells of Bordetella pertussis after inactivation (inactivated vaccine). However, localized reactions at the site of vaccination and side reactions, such as fever, have been reported, creating a social urge to solve this problem. To solve this problem, there have been a large number of attempts of using protective components separated from Bordetella pertussis as vaccine. For example, acellular pertussis vaccine (ACP vaccine), prepared by extracting protective proteins, such as pertussis toxin (PT), pertussis filamentous hemagglutinin (FHA), pertactin (PRN, 69K-OMP) and pertussis fimbriae (FIM), from Bordetella pertussis cells, and removing endotoxin (ET), is being put into practical application, but is not fully satisfactory, due to the drawbacks described below.

Pertussis toxin (PT), pertussis filamentous hemagglutinin (FHA), pertactin (PRN, 69K-OMP) and pertussis fimbriae (FIM), all protective components of Bordetella pertussis already in practical application with validated efficacy, are separated by respective methods.

Pertussis toxin (PT) can be separated by affinity chromatography using human haptoglobin as a ligand [Biochimica et Biophysica Acta, Vol. 580, p. 175 (1979)]. However, human haptoglobin can be contaminated with hepatitis virus, because it is collected from human blood; the same applies when animal sera are used. Another available method is affinity chromatography using denatured ceruloplasmin as a ligand (Japanese Patent Unexamined Publication No. 62135/1987). Although this method is free of the problem of viral contamination, some problems arise, including vaccine contamination with ceruloplasmin and the high toxicity and potential body retention of sodium thiocyanate and other eluents having protein-denaturing effect.

As for pertussis filamentous hemagglutinin (FHA), a purification method using hydroxyapatite gel is available [Infection and Immunity, Vol. 41, p. 313 (1983) and EP-A-231083, EP-A-427462, EP-A-462534; Japanese Patent Unexamined Publication Nos. 234031/1987, 169893/1992, 368337/1993). However, it takes a long time for column operation, and is uneconomical due to the high cost of hydroxyapatite.

As for pertactin (PRN, 69K-OMP), affinity chromatography using a mouse serum as a ligand is available [Infection and Immunity, Vol. 56, p. 3189 (1988)], but has the same drawbacks as above.

As for pertussis fimbriae (FIM), Bordetella pertussis cell extract is purified by salting-out with ammonium sulfate and magnesium chloride [Infection and Immunity, Vol. 48, p. 442 (1985)], but this method is poor in vaccine production efficiency due to low yield.

There is a method of preparing Gram-negative bacterial vaccine by adosorbing with the aluminum hydroxide gel (WO 93/10216). This method needs the large amount of the aluminum hydroxide gel, which adsorbs both the protective components and the endotoxin from Gram-negative bacteria. The vaccine obtained by the method of W093/10216 has a danger of side effects, such as fever and endotoxinshock, by the endotoxin released into the body because of the diluted vaccines.

As for the pertussis vaccine production included as the components mixture without separating each protective component from Bordetella pertussis, a method of using calcium phosphate gel is available (EP-A-291968, Japanese Patent Unexamined Publication No. 52726/1989). However, this method formed the calcium phosphate in the presence of a 1M sodium chloride does not absorb the protective components.

As stated above, totally different purification methods must be used to separate the respective protective components of Bordetella pertussis. This approach is unsuitable to large-scale vaccine production due to painstaking operation, and difficult to apply practically. Moreover, the customary methods of separating protective components disclosed in prior art have some problems that materials or reagents have pathogenicity or toxity.

### Disclosure of Invention

Against the background described above, the present inventors investigated methods of efficiently separating protective components of Bordetella pertussis, and found that protective components of Bordetella pertussis can be efficiently separated from Bordetella pertussis culture on the basis of differences in adsorbability to calcium phosphate gel formed by adding calcium ions to the Bordetella pertussis culture in the presence of excess phosphate ions. The inventors made further investigation based on this finding, and the efficient and safe method of separating the protective components combined with the calcium phosphate gel treatment and elution by salt and heating was developed the present invention. Accordingly, the present invention relates to:
(1) A method of separating at least one member of the group consisting of pertussis filamentous hemagglutinin (FHA), pertactin (PRN, 69K-OMP), pertussis fimbriae (FIM), and pertussis toxin (PT) by bringing a Bordetella pertussis culture into contact with calcium phosphate gel which is formed by adding calcium ions to the culture in the presence of phosphate ions.
(2) A method of separating at least one member of the group consisting of pertussis filamentous hemagglutinin (FHA), pertactin (PRN, 69K-OMP), pertussis fimbriae (FIM) and pertussis toxin (PT) by separating a Bordetella pertussis culture into cells and culture liquid, and carrying out at least one of processes (A), (B), (C) and (D):
   (A) a process in which the separated cells are eluted with a salt solution, and pertussis filamentous hemagglutinin (FHA) is separated by bringing the eluted solution into contact with calcium phosphate gel of the above item (1),
   (B) a process in which the cell residue resulting from the elution treatment of the above process (A) is heated in the presence of a salt solution and brought into contact with calcium phosphate gel, and pertactin (PRN, 69K-OMP) is separated by bringing the eluted solution into contact with calcium phosphate gel of the above item (1),
   (C) a process in which the cell residue resulting from the elution treatment of the above process (A) is heated in the presence of a salt solution, the supernatant is brought into contact with calcium phosphate gel and eluted with a salt solution, and pertussis fimbriae (FIM) is separated by bringing the eluted solution into contact with calcium phosphate gel of the above item (1),
   (D) a process in which the culture or the separated culture liquid is brought into contact with calcium phosphate gel of the above item (1), and pertussis toxin (PT) is separated from the supernatant.
(3) The separation method of the above item (2), wherein the supernatant is brought into contact with calcium phosphate gel and eluted with a salt solution to separate pertussis filamentous hemagglutinin (FHA) in process (A).
(4) The separation method of the above item (2), wherein the supernatant after being brought into contact with calcium phosphate gel is brought into contact with ion exchange gel to separate pertactin (PRN, 69K-OMP) in process (B).
(5) The separation method of the above item (2), wherein the supernatant is brought into contact with calcium phosphate gel and removed, and the resulting residue is eluted with a salt solution to separate pertussis fimbriae (FIM) in process (C).
(6) The separation method of the above item (2), wherein the supernatant is brought into contact with ion exchange gel to separate pertussis toxin (PT) in process (D).
(7) The separation method of the above item (2), wherein the salt solution used in processes (A) and (C) is a buffer containing an alkali metal salt.
(8) The separation method of the above item (7), wherein the salt solution is a buffer containing 0.01-1.0 M sodium chloride.
(9) The separation method of the above item (1) or (2), wherein the calcium phosphate gel is formed by adding calcium ions to the culture or the supernatant of pH 7-9 in the presence of phosphate ions.
(10) The separation method of the above item (9), wherein the equivalent ratio of phosphate ions and calcium ions is 1.25-30 equivalents of phosphate ions per equivalent of calcium ions.
(11) The separation method of the above item (9), wherein the calcium phosphate gel is formed by adding calcium acetate, as a calcium ion source, at 0.1-2 w/v% in the presence of a 0.05-0.1 M phosphate buffer.
(12) The separation method of the above item (1) or (2), wherein at least one member of the group consisting of pertussis toxin (PT), pertussis filamentous hemagglutinin (FHA), pertactin (PRN, 69K-OMP) and pertussis fimbriae (FIM) is separated, after which endotoxin is removed by adsorption to aluminum hydroxide gel in the presence of ammonium sulfate.
(13) The separation method of the above item (1) or (2), wherein at least one member of the group consisting of pertussis toxin (PT), pertussis filamentous hemagglutinin (FHA), pertactin (PRN, 69K-OMP) and pertussis fimbriae (FIM) is separated, after which endotoxin is removed by zonal centrifugation.
(14) A pertussis vaccine wherein the components PT:FHA:FIM are admixed in a ratio of 4-6:8-10:1.
(15) A pertussis vaccine wherein the components PT:FHA:PRN:FIM are admixed in a ratio of 2-6:4-10:1-2:1.

### Best Mode for Carrying Out the Invention

The Bordetella pertussis strain used for the present invention is not subject to limitation, as long as it is capable of producing one or more than one member of the group consisting of pertussis filamentous hemagglutinin (FHA), pertactin (PRN, 69K-OMP), pertussis fimbriae (FIM) and pertussis toxin (PT), all protective components of Bordetella pertussis. Useful strains include known strains, such as Bordetella pertussis Tohama phase I strain [Infection and Immunity, Vol. 6, p. 89, (1972)] (maintained at the National Institute of Health, Ministry of Social Welfare, Tokyo, Japan (NIHJ 1052), deposited under accession number IFO 14073 at the Institute for Fermentation, Osaka since August 13, 1980), Bordetella pertussis Yamaguchi phase I strain, Bordetella pertussis phase I strain 18-323 and Bordetella pertussis phase I strain 165, with preference given to Bordetella pertussis Tohama phase I strain (IFO 14073) from the viewpoint of productivity. Bordetella pertussis can be cultured by known methods. Useful media include known basal media, such as Cohen-Wheeler medium, Stainer-Scholte medium and other liquid media, with preference given to Stainer-Scholte medium. The solution containing protective components and endotoxin (ET) may be a culture obtained by stationary culture or tank culture. In the present invention, the culture means cultured cells or culture liquid resulting from incubating said Bordetella pertussis. And the present invention, the supernatant means the culture liquid or the supernatant resulting from heating the cells in the presence of a salt solution or eluting with a salt solution from the calcium phosphate gel adsorbed the protective components as described below. The cells include the culture cells and the cell residue. In the present invention, the method used to separate a Bordetella pertussis culture into cells and culture supernatant may be a known method, such as centrifugation or filtration.

The calcium phosphate gel used for the present invention is not a ready-made gel, but preferably calcium phosphate gel formed in a culture or a supernatant to be treated by adding calcium ions to them in the presence of excess phosphate ions (may referred to as the in-side gel forming method). Although prepared calcium phosphate gel is commercially available as hydroxyapatite gel, comparison with the former method by using the ready-made hydroxyapatite gel mentioned above (may referred to as the out-side gel forming method), with the present method by using calcium phosphate gel is higher in both adsorption efficiency for pertussis filamentous hemagglutinin (FHA) and pertussis fimbriae (FIM) and recovery efficiency of them, as shown hereafter. Moreover, the calcium phosphate gel used in the present invention is better in operational efficiency because of the absence of gel pretreatment and regeneration process, and more advantageous in cost. Furthermore, each of protective components of Bordetella pertussis can be selectively absorbed to the calcium phosphate gel by properly selecting the ratio of phosphate ions to calcium ions. If the the culture or the supernatant to be treated with calcium phosphate gel, does not contain a sufficient amount of phosphate ions, a phosphate buffer of appropriate concentration is added to provide phosphate ions before addition of calcium ions. For example, by adding 1 M phosphate buffer, the final phosphate ion concentration is adjusted to 0.02-0.2 M, preferably 0.05-0.1 M.

The calcium ion source added is exemplified by soluble calcium salts, such as calcium acetate, calcium chloride and calcium nitrate, with preference given to calcium ions derived from calcium acetate. Concerning the ratio of phosphate ions and calcium ions, it is preferable that phosphate ions be in excess, in comparison with calcium ions. The ratio can be properly selected in each case of the protective components of Bordetella pertussis, as mentioned hereafter.

In process (A) above, pertussis filamentous hemagglutinin (FHA) is separated as follows: After the culture liquid, i.e. the culture supernatant, is removed from a Bordetella pertussis culture by a known method, such as centrifugation or filtration, a one-tenth to one-twentieth volume (relative to the amount of culture broth) (corresponding to a final cell concentration of 50-100 billion cells/ml) of a salt solution is added to the cells to elute the hemagglutinin. In this case, the salt solution used is preferably a buffer supplemented with an alkali metal salt or an alkaline earth metal salt, specifically a 0.04-0.08 M phosphate buffer supplemented with a 0.25-1.0 M alkali metal salt or alkaline earth metal salt, with greater preference given to a 0.05 M phosphate buffer supplemented with a 0.5-1.0 M alkali metal salt. The alkali metal salt or alkaline earth metal salt added to the buffer is exemplified by sodium chloride, potassium chloride and magnesium chloride. For example, it is preferable to elute the hemagglutinin by adding a one-tenth to one-twentieth volume (relative to the amount of culture broth) of a 0.04-0.08 M phosphate buffer (pH 7-9) supplemented with 0.5-1.0 M sodium chloride, more preferably a 0.05 M phosphate buffer (pH 8) supplemented with 1 M sodium chloride, to the cells collected, followed by gentle stirring at 4°C to room temperature, preferably 8-15°C, for 1-60 minutes, preferably 1-30 minutes, and standing for 1-2 days. The solution containing the eluted pertussis filamentous hemagglutinin (FHA) is then subjected to a known method, such as centrifugation or filtration, to recover the supernatant (the cell residue obtained at the same time by this treatment is used to isolate pertactin (PRN, 69K-OMP) and pertussis fimbriae (FIM)). The thus-obtained supernatant is then brought into contact with calcium phosphate gel.

Concerning the ratio of phosphate ions and calcium ions, it is preferable that phosphate ions be in excess, in comparison with calcium ions. For example, the equivalent ratio of these ions is preferably 1.25-30 equivalents, more preferably 1.5-7.5 equivalents of phosphate ions per equivalent of calcium ions. This quantitative ratio can be expressed in molar ratio as 0.8-20 M of phosphate ions to 1 M of calcium ions, more preferably 1-5 M of phosphate ions to 1 M of calcium ions. For example, to a solution (pH 7-9) containing phosphate ions at a concentration within the above-described concentration range (0.02-0.2 M, preferably 0.05-0.1 M), a calcium salt is added to a final concentration of 4-70 mM, preferably 8-50 mM (e.g., calcium acetate added to a final concentration of 0.1-0.8 w/v%, preferably 0.2-0.6 w/v%), followed by gentle reaction at 4°C to room temperature, preferably 8-15°C, for 1 to 4 hours, preferably 1 to 2 hours, to form calcium phosphate gel.

Although pertussis filamentous hemagglutinin (FHA) is adsorbed to the calcium acetate gel added, provided that the amount of calcium phosphate gel added to a final concentration exceeding 0.8 w/v%, it is preferable to add the calcium acetate gel in an amount such that the final concentration falls within the above concentration range, for selectively adsorbing pertussis filamentous hemagglutinin (FHA) only. After completion of the reaction, the supernatant is removed by a known method, such as centrifugation or filtration; the resulting gel precipitate is collected. To this precipitate, a one-tenth to one-twentieth volume (relative to the amount of culture broth) of a salt solution is added, to elute the pertussis filamentous hemagglutinin (FHA). In this case, the salt solution used may be the same salt solution as used to elute pertussis filamentous hemagglutinin (FHA) from the above-described cells. It is preferable to add a one-tenth to one-twentieth volume of 0.05-0.1 M phosphate buffer (pH 7-9) supplemented with 1-2 M sodium chloride, more preferably 0.1 M phosphate buffer (pH 8) supplemented with 1-1.5 M sodium chloride, to the above-described gel precipitate, followed by gentle stirring at 4°C to room temperature for 1 to 2 hours, to elute the hemagglutinin. After completion of the stirring, the precipitate is removed by a known method, such as centrifugation or filtration, to recover pertussis filamentous hemagglutinin (FHA) in the supernatant. The supernatant, if necessary, can be concentrated and desalinized, by ammonium sulfate salting-out or using an ultrafiltration membrane. By subjecting the supernatant obtained by the above-described treatment to the aluminum hydroxide gel treatment or zonal centrifugation described below, pertussis filamentous hemagglutinin (FHA) having endotoxin selectively removed can be separated with substantially no loss.

In process (B) or (C) above, pertactin (PRN, 69K-OMP) and pertussis fimbriae (FIM) are separated as follows: The cell residue resulting from elution of the solution containing pertussis filamentous hemagglutinin (FHA) is heated in the presence of a one-tenth to one-twentieth volume (relative to the amount of culture broth) (corresponding to a final cell concentration of 500-100 billion cells/ml) of a salt solution to extract the pertactin (PRN, 69K-OMP) and pertussis fimbriae (FIM). In this case, the salt solution used may be the same as used in process (A) above. However, it is preferable to use a one-tenth to one-twentieth volume (relative to the amount of culture broth) of 0.01-0.05 M phosphate buffer (pH 7-9) supplemented with 0.15-0.25 M sodium chloride, with greater preference given to 0.01 M phosphate buffer (pH 7) supplemented with 0.15-0.25 M sodium chloride. It is preferable that heating be achieved in warm water at 40-80°C, preferably 50-60°C, for 60 to 120 minutes, preferably 80 to 90 minutes. The heated extracted pertactin (PRN, 69K-OMP) and pertussis fimbriae (FIM) are recovered in the supernatant by a known method, such as centrifugation or filtration. The thus-obtained supernatant is then brought into contact with calcium phosphate gel. In this case, calcium phosphate gel treatment can be performed in accordance with process (A) above; however, it is preferable to perform it within the following concentration range. For example, to a solution (pH 7-9) containing phosphate ions, adjusted as necessary to a final phosphate ion concentration of 0.05-0.1 M, preferably 0.1 M, by adding a 1 M phosphate buffer, or the like, a calcium salt is added to a final concentration of 40-180 mM, preferably 55-150 mM (e.g., calcium acetate added to a final concentration of 1-2 w/v%, preferably 1.3-1.7 w/v%), followed by gentle reaction at 4°C to room temperature, preferably 8-15°C, for 1 to 4 hours, preferably 1 to 2 hours, to form calcium phosphate gel. After completion of the reaction, the resulting precipitate and supernatant are separated from each other by a known separation method, such as filtration or centrifugation, to recover pertactin (PRN, 69K-OMP) in the supernatant and pertussis fimbriae (FIM) in the gel residue, with substantially no loss.

The crudely purified pertactin (PRN, 69K-OMP) obtained by the above-described treatment can be further purified by a known method, preferably by ion exchange gel treatment; it is preferable that the crudely purified pertactin (PRN, 69K-OMP) be previously concentrated and desalinized by ammonium sulfate salting-out or using an ultrafiltration membrane. In the present invention, useful ion exchange gels include anion exchange gel and cation exchange gel, with preference given to cation exchange gel. Contact with ion exchange gel may be achieved by the column chromatography method or the batch method. By this treatment, impurities, i.e., substances other than pertactin (PRN, 69K-OMP) in the crudely purified pertactin (PRN, 69K-OMP), are adsorbed; the effluent is collected to yield a solution containing pertactin (PRN, 69K-OMP). In the column chromatography method, the column is packed with ion exchange gel, through which the starting material, i.e., crudely purified pertactin (PRN, 69K-OMP), is passed at a flow rate of 100-500 ml/cm²/hr. In the batch method, crudely purified pertactin (PRN, 69K-OMP) is placed in a container, to which ion exchange gel is added directly, followed by stirring for about 30 minutes to 3 hours, preferably about 1 hour, to adsorb impurities, i.e., substances other than pertactin (PRN, 69K-OMP). Such impurity adsorption is achieved using a buffer of a pH value of 5.0-8.0 and an electroconductivity of 100-300 umho (0.1-0.3 mS), e.g., a 0.01-0.02 M phosphate buffer (pH 5.5-6.0). By subjecting the supernatant obtained by the above-described treatment to the aluminum hydroxide gel treatment or zonal centrifugation treatment described below, pertactin (PRN, 69K-OMP) having endotoxin removed can be separated with substantially no loss.

To the gel residue containing crude pertussis fimbriae (FIM) obtained by the above-described treatment, a one-tenth to one-twentieth volume (relative to the amount of culture broth) of a salt solution is added, to elute the pertussis fimbriae (FIM). In this case as well, the salt solution may be the same as used in process (A) above. For example, it is preferable to add a one-tenth to one-twentieth volume (relative to the amount of culture broth) of a 0.05-0.1 M phosphate buffer (pH 7-9) supplemented with 1-2 M sodium chloride, preferably a 0.1 M phosphate buffer (pH 8) supplemented with 1-1.5 M sodium chloride, followed by gentle stirring at 4°C to room temperature for 1 to 2 hours, to elute the pertussis fimbriae (FIM). After completion of the stirring, the precipitate is removed by a known method, such as centrifugation or filtration, to recover pertussis fimbriae (FIM) in the supernatant. By subjecting the supernatant obtained by the above-described treatment to the above-described aluminum hydroxide gel treatment or zonal centrifugation treatment, pertussis fimbriae (FIM) having endotoxin selectively removed can be separated with substantially no loss.

In process (D) above, pertussis toxin (PT) is separated as follows: Although a Bordetella pertussis culture can be used without separation into cultured cells and culture supernatant in this process, it is preferable in respect of efficiency to recover the supernatant from the Bordetella pertussis culture by a known method, such as centrifugation or filtration, concentrate the supernatant about 10-20 fold using an ultrafiltration membrane, or the like, and collect the supernatant by centrifugation or another method before this process. This supernatant is then brought into contact with calcium phosphate gel. In this case, calcium phosphate gel treatment can be carried out in the same manner as in process (A) above, but it is preferable to carried out this treatment within the following concentration range. For example, to a solution (pH 7-9) containing phosphate ions, adjusted as necessary to a final phosphate ion concentration of 0.05-0.1 M, preferably 0.1 M, by adding a 1 M phosphate buffer, or the like, a calcium salt is added to a final concentration of 40-180 mM, preferably 55-150 mM (e.g., calcium acetate added to a final concentration of 1-2 w/v%, preferably 1.3-1.7 w/v%), followed by gentle reaction at 4°C to room temperature, preferably 8-15°C, for 1 to 4 hours, preferably 1 to 2 hours, to form calcium phosphate gel. After completion of the reaction, the resulting precipitate and supernatant are separated from each other by a known method, such as centrifugation or filtration, to recover pertussis toxin (PT) in the supernatant with substantially no loss. The crudely purified pertussis toxin (PT) obtained by the above-described treatment is further purified by ion exchange gel treatment; it is preferable that the crudely purified pertussis toxin (PT) be previously concentrated and desalted by ammonium sulfate salting-out or using an ultrafiltration membrane. The ion exchange gel used here is exemplified by anion exchange gel and cation exchange gel, with preference given to cation exchange gel. Contact with ion exchange gel may be achieved by the column chromatography method or the batch method. By this treatment, pertussis toxin (PT) in the crudely purified pertussis toxin (PT) is adsorbed to the gel, followed by washing with an appropriate buffer to elute and remove impurities, after which pertussis toxin (PT) is eluted and isolated with a buffer of appropriate pH and ionic strength. In the column chromatography method, the column is packed with ion exchange gel, through which the starting material, i.e., crudely purified pertussis toxin (PT), is passed at a flow rate of 100-500 ml/cm²/hr to cause toxin adsorption. In the batch method, the crudely purified pertussis toxin (PT) is placed in a container, to which ion exchange gel is added directly, followed by stirring for about 30 minutes to 3 hours, preferably about 1 hour, to cause toxin adsorption. Such adsorption of the crudely purified pertussis toxin (PT) is achieved using a buffer of a pH level of 5.0-6.0 and an electroconductivity of 100-300 umho (0.1-0.3 mS), e.g., a 0.01-0.02 M phosphate buffer (pH 5.5-6.0). Elution from the ion exchange gel to which the pertussis toxin (PT) has been adsorbed can be achieved using a buffer of a pH level of 7.0-7.5 and an electroconductivity of 1,000-2,000 umho (1-2 mS), e.g., a 0.1-0.2 M phosphate buffer (pH 7.0-7.5). By subjecting the eluate obtained by the above-described treatment to the aluminum hydroxide gel treatment or zonal centrifugation treatment described below, pertussis toxin (PT) having endotoxin selectively removed can be separated with substantially no loss.

In the present invention, the aluminum hydroxide gel treatment for endotoxin removal is carried out to adsorb only the endotoxin selectively by bringing the subject into contact with previously prepared aluminum hydroxide gel in the presence of ammonium sulfate. But, the aluminum hydroxide gel, whose amount to be used is less than one-tenth of that used in W093/10216, hardly absorbs any amounts of protective components of Bordetella pertussis. It is normally preferable that this treatment be carried out after concentration by a known method, such as ammonium sulfate salting-out or an ultrafiltration membrane method. Aluminum ions useful for the previously prepared aluminum hydroxide gel include those of soluble aluminum compounds, such as aluminum sulfate and aluminum chloride, with preference given to the aluminum ions of aluminum chloride. It is preferable that aluminum hydroxide gel be prepared by adding a 2 M sodium hydroxide solution to a 25-190 mM aluminum salt solution (e.g., 0.9-4.5% aluminum chloride solution) to a pH level of 7.0-7.5, followed by gentle reaction at 4°C to room temperature for 1 to 3 hours, to form the desired aluminum hydroxide gel. The aluminum hydroxide gel obtained by the above-described treatment is then treated to recover the resulting gel precipitate by a known method, such as filtration or centrifugation, to remove free aluminum ions after completion of the reaction. The protective component of Bordetella pertussis concentrated by a known method, such as ammonium sulfate salting-out, is recovered by centrifugation; the precipitate is dissolved in a 0.25 M phosphate buffer (pH 7.0-7.5) supplemented with 0.25 M sodium chloride. To this protective component of Bordetella pertussis, a saturated ammonium sulfate solution is added to a final concentration of 2.0-8.0 v/v%, followed by addition of previously prepared, recovered aluminum hydroxide gel to a final concentration of 0.1-1.0 mg/ml, preferably 0.2-0.5 mg/ml, and gentle reaction at 4°C to room temperature for 30 minutes to 1 hour. After completion of the reaction, the aluminum hydroxide gel is removed by a known method, such as filtration or centrifugation, to separate the protective component of Bordetella pertussis having endotoxin removed, with substantially no loss.

In the present invention, zonal centrifugation treatment is carried out to remove endotoxin, and is preferably carried out after concentration by a known method, such as ammonium sulfate salting-out. Zonal centrifugation methods include sucrose density gradient centrifugation, cesium chloride density gradient centrifugation and potassium tartrate density gradient centrifugation, with preference given to sucrose density gradient centrifugation. For example, when sucrose density gradient centrifugation is carried out on a sucrose density gradient of 0-30 w/v% at an Rₘₐₓ of 60,000 to 122,000 G for about 10 to 24 hours, the protective component of Bordetella pertussis having endotoxin removed can be separated.

PT is detoxified by using a conventional detoxification technique as described in British Journal of Experimental Pathslogy, vol. 44, p. 177, (1963). FHA, PRN and FIM may be inactivated, for example, by the method as described in Japanese Patent Unexamined Publication No. 52726/1989. An improved purified pertussis component vaccine which is superior to a known pertussis vaccine can be produced by blending in any desireded ratio of protective components of Bordetella pertussis obtained by the method of present invention. Namely, it is not possible to change the ratio of each component which is stable in whole cell or co-purified acellular vaccine without obtaining furified component respectively, while an antigen ratio can be chosen in the method of present invention which gives the optimal which gives the optimal response in humans as a pertussis vaccine since each component is efficiently purified in the present invention. The purified pertussis component vaccine is desirable to blend the protective components in as little amount of total protein as possible and in a way of giving more effective immunogenicity. The purified pertussis component vaccine of the present invention preferably includes all of three components, i.e. FHA, FIM and PT, and may also include other pharmaceutically acceptable components such as PRN which does not give undesired side effects.

When blending these components to produce a purified pertussis component vaccine of the present invention, the ratio of it may be examplified in Examples metioned hereinafter. The component vaccine of the present invention has a PT:FHA:FIM ratio of approximate 4-6:8-10:1, preferably 5-6:8-10:1, and comprise, for example, 20-30 µg-protein/ml of PT, 40-50 µg-protein/ml of FHA and 5-10 µg-protein/ml of FIM, preferably 25-30 µg-protein/ml of PT, 40-50 µg-protein/ml of FHA and 5 µg-protein/ml of FIM. The component vaccine mentioned above may further include 5-10 µg-protein/ml of PRN, and has a PT:FHA:PRN:PT ratio of 2-6:4-10:1-2:1, preferably 5-6:8-10:2:1. Namely, it preferably comprise 25-30 µg-protein/ml of PT, 40-50 µg-protein/ml of FHA, 10 µg-protein/ml of PRN and 5 µg-protein/ml of FIM.

The above-described effect of the present invention can be summarized as follows: The method of the present invention is characterized by the use of the same means of purification for all subject protective components of Bordetella pertussis. This obviates the necessity of different painstaking procedures for the respective components as in prior art methods, thus permitting component purification with high efficiency and high recovery rate, an aspect very advantageous for industrial production. In addition, the endotoxin content, as determined by the Limulus test, is not more than 1 ng per 100 µg total protein, for all protective components of Bordetella pertussis obtained by the present invention, providing very high practical value. It is also possible to produce an improved purified pertussis component vaccine comprising an effective combination of pertussis filamentous hemagglutinin (FHA), pertactin (PRN, 69K-OMP), pertussis fimbriae (FIM) and pertussis toxin (PT).

### Examples

The present invention is hereinafter described in more detail by means of the following working examples and reference examples. In the following description, pertussis toxin (PT), pertussis filamentous hemagglutinin (FHA), pertactin (PRN, 69K-OMP), pertussis fimbriae (FIM) and endotoxin are also referred to as PT, FHA, 69K-OMP, FIM and ET, respectively.

### Example 1

Bordetella pertussis Tohama phase I strain was cultured to a final concentration of 2 billion cells/ml by Roux bottle stationary culture (450 ml, 35°C, 5 days) and tank agitating culture (40 1, 35°C, 2 days) using Stainer-Scholte medium, to yield a Bordetella pertussis culture.

The cell culture was concentrated to a one-tenth volume using an ultrafiltration membrane, after which it was centrifuged to separate the supernatant and cells. To the supernatant, a 1 M phosphate buffer (pH 8.0) was added to a final concentration of 0.1 M, followed by addition of an calcium acetate solution to a final concentration of 1.6 w/v% and stirring at room temperature for 1 hour. This calcium phosphate gel solution was filtered. The resulting filtrate was concentrated and desalted to an electroconductivity of 200 Ohm using an ultrafiltration membrane, passed through a sulfopropyl cation exchange chromatography column (produced by Tosoh Corporation), washed with a 0.01 M phosphate buffer (pH 6.0), and eluted with a 0.1 M phosphate buffer (pH 7.0), to yield pertussis toxin (PT). Next, cells were dispersed in a one-tenth volume (relative to the amount of culture broth) of a 0.05 M phosphate buffer (pH 8.0) supplemented with 1 M sodium chloride, followed by centrifugation to yield the supernatant and cells. To the supernatant, a calcium acetate solution was added to a final concentration of 0.5 w/v%, followed by stirring at room temperature for 1 hour. This calcium phosphate gel solution was filtered; the resulting gel layer was collected. The gel layer was eluted with a 0.1 M phosphate buffer (pH 8.0) supplemented with 1 M sodium chloride to yield a solution containing pertussis filamentous hemagglutinin (FHA). Separately, cells were dispersed in a one-tenth volume (relative to the amount of culture broth) of a 0.01 M phosphate buffer (pH 7.0) supplemented with 0.15 M sodium chloride, after which it was heated in 60°C warm water for 90 minutes, followed by centrifugation to yield the supernatant. To the supernatant, a 1 M phosphate buffer (pH 8.0) was added to a final concentration of 0.1 M, after which a calcium acetate solution was added to a final concentration of 1.6 w/v%, followed by stirring at room temperature for 1 hour. This calcium phosphate gel solution was filtered; the filtrate and the gel layer were collected. The filtrate was concentrated and desalted to an electroconductivity of 200 Ohm using an ultrafiltration membrane and passed through a sulfopropyl cation exchange chromatography column (produced by Tosoh Corporation); the effluent was collected to yield a solution containing pertactin (PRN, 69K-OMP). Separately, the gel layer was eluted with a 0.1 M phosphate buffer (pH 8.0) supplemented with 1 M sodium chloride to yield a solution containing pertussis fimbriae (FIM).

Control sample was prepared as follows: Ammonium sulfate was added at 220 g per liter of culture broth, followed by sufficient stirring. After being kept standing at 4°C for about 14 days, the mixture was centrifuged; the supernatant was discarded, and the precipitate was collected. To the precipitate thus obtained, a one-tenth volume (relative to the amount of culture broth) of a 0.05 M phosphate buffer (pH 8.0) supplemented with 1 M sodium chloride was added, followed by sufficient stirring. After being kept standing at 4°C for 4 days, the mixture was again centrifuged; the supernatant was collected to yield a solution containing pertussis toxin (PT), pertussis filamentous hemagglutinin (FHA), pertactin (PRN, 69K-OMP) or pertussis fimbriae (FIM).

The pertussis toxin (PT), pertussis filamentous hemagglutinin (FHA), pertactin (PRN, 69K-OMP) or pertussis fimbriae (FIM) content in each sample was determined by ELISA, with purified products of pertussis toxin (PT), pertussis filamentous hemagglutinin (FHA), pertactin (PRN, 69K-OMP) and pertussis fimbriae (FIM) as references. Results are expressed in µg protein/ml unit.

Protein content determination: Protein precipitated with heated trichloroacetic acid was quantitated by the Lowry method, with bovine serum albumin (Fraction V, produced by Wako Pure Chemical Industries) as a reference. Results are expressed in µg protein/ml unit.

The results for Roux bottle culture broth and those for tank culture broth are shown in Tables 1 and 2, respectively.

**Table 1**

| Sample | Active Ingredient Protein Content (µg protein/ml) | Recovery*(%) | Total Protein Content (µg protein/ml) | Purity (%) (active ingredient protein content/total protein content) |
|---|---|---|---|---|
| PT | 2656.8 | 90.0 | 2662.1 | 99.8 |
| FHA | 9161.7 | 85.0 | 9339.1 | 98.1 |
| FIM | 474.7 | 244.6 | 495.5 | 95.8 |
| 69K-OMP | 3683.8 | 244.6 | 3607.2 | 102.1 |

| | | | | |
|---|---|---|---|---|
| * Each figure represents a percent value relative to the control group. | | | | |

**Table 2**

| Sample | Active Ingredient Protein Content (µg protein/ml) | Recovery* (%) | Total Protein Content (µg protein/ml) | Purity (%) (active ingredient protein content/total protein content) |
|---|---|---|---|---|
| PT | 3242.2 | 78.9 | 3359.8 | 96.5 |
| FHA | 9527.0 | 98.0 | 9752.9 | 97.7 |
| FIM | 675.0 | 1184.0 | 714.7 | 95.0 |
| 69K-OMP | 4333.5 | 2364.0 | 4505.1 | 96.3 |

| | | | | |
|---|---|---|---|---|
| * Each figure represents a percent value relative to the control group. | | | | |

It is evident from these figures that each protective component was efficiently isolated, and that in the case of tank culture broths, pertactin (PRN, 69K-OMP) and pertussis fimbriae (FIM), both produced at low productivity in the case of Roux bottle culture broths, were recovered in large amounts.

### Reference Example 1

To a control solution prepared by the method described in Example 1, calcium acetate was added to a final concentration of 0.5 w/v%, followed by stirring at room temperature for 1 hour. To the filtrate obtained by filtering this calcium solution, a half amount of a saturated ammonium sulfate solution was added; the mixture was kept standing at 4°C for 7 days. This ammonium sulfate salting-out product was centrifuged; the resulting precipitate was collected and resuspended in a 0.025 M phosphate buffer (pH 7.0) supplemented with 0.25 M sodium chloride to yield a starting material. To this starting material, aluminum hydroxide gel, previously prepared to a final concentration of 0.4 mg/ml, was added; to the aluminum hydroxide gel recovered by centrifugation, ammonium sulfate was added to a final concentration of 0, 2, 4 or 8 w/v%, followed by gentle stirring at room temperature for 30 minutes. After completion of the reaction, the aluminum hydroxide gel was removed by centrifugation to separate the supernatant. Each supernatant was assayed for hemagglutination activity and endotoxin content by the methods described below. The results are shown in Table 3.

Determination of hemagglutination activity: After the sample was serially diluted 2 folds with a 0.01 M phosphate buffered saline, 0.6 v/v% chick immobilized red blood cells were added to cause hemagglutination. The maximum dilution rate of each sample showing hemagglutination was taken as the hemagglutinin titer HA. Determination of endotoxin (ET) content: Using *Escherichia coli* (Difico 055-B5) as a reference strain, ET content was determined by the Limulus test (Wako Pure Chemical kit). Results are expressed in ng/ml unit. It is evident from Table 3 that endotoxin can be selectively removed, without active ingredient loss, by treating the sample with previously prepared aluminum hydroxide gel in the presence of ammonium sulfate.

**Table 3**

| Amount of Ammonium Sulfate Added (w/v%) | Endotoxin Content (ng/ml) | HA Value (HAU/ml) | HA Recovery Rate* (%) |
|---|---|---|---|
| 0 | 15.8 | 16000 | 50.0 |
| 2 | 11.1 | 32000 | 100.0 |
| 4 | <9.0 | 32000 | 100.0 |
| 8 | <9.0 | 24000 | 75.0 |

| | | | |
|---|---|---|---|
| * Each figure for endotoxin removal rate or HA recovery rate is a percent value relative to the pretreatment value. | | | |

### Example 2

To each of pertussis toxin (PT), pertussis filamentous hemagglutinin (FHA), pertactin (PRN, 69K-OMP) and pertussis fimbriae (FIM) as obtained in Example 1, a half amount of a saturated ammonium sulfate solution was added, followed by sufficient stirring. After being kept standing at 4°C for 1 week, the mixture was again centrifuged; the resulting precipitate was collected.

This precipitate was dissolved in a 0.025 M phosphate buffer (pH 7.0) supplemented with 0.25 M sodium chloride to yield a solution of pertussis toxin (PT), pertussis filamentous hemagglutinin (FHA), pertactin (PRN, 69K-OMP) or pertussis fimbriae (FIM). To each solution, a saturated ammonium sulfate solution was added to a final concentration of 4.0 v/v%. To this mixture, previously prepared, recovered aluminum hydroxide gel was added to a final concentration of 0.4 mg/ml, followed by gentle stirring for 30 minutes at room temperature. After completion of the reaction, the aluminum hydroxide gel was removed by centrifugation to yield pertussis toxin (PT), pertussis filamentous hemagglutinin (FHA), pertactin (PRN, 69K-OMP) and pertussis fimbriae (FIM).

Pertussis toxin (PT) content, pertussis filamentous hemagglutinin (FHA) content, pertactin (PRN, 69K-OMP) content and pertussis fimbriae (FIM) content were determined in the same manner as in Example 1; and endotoxin content, in the same manner as in Reference Example 1. The results are shown in Table 4.

**Table 4**

| Sample | Endotoxin Content (ng/100 µg protein) | Active Ingredient Protein Content (µg protein/ml) | Recovery Rate*(%) |
|---|---|---|---|
| PT | 0.01 | 2999.0 | 82.5 |
| FHA | 0.11 | 9060.2 | 95.1 |
| FIM | 0.54 | 478.6 | 70.9 |
| 69K-OMP | 0.08 | 3505.8 | 80.9 |

| | | | |
|---|---|---|---|
| * Each figure for recovery rate represents a percent ratio relative to the pretreatment value. | | | |

It is evident from this table that endotoxin was selectively removed, with substantially no loss of any protective component, the endotoxin content per 100 µg protein/ml being not more than 1 ng/ml for all components.

### Example 3

To each of pertussis toxin (PT), pertussis filamentous hemagglutinin (FHA), pertactin (PRN, 69K-OMP) and pertussis fimbriae (FIM) as obtained in Example 1, a half amount of a saturated ammonium sulfate solution was added, followed by sufficient stirring. After being kept standing at 4°C for 1 week, the mixture was again centrifuged; the resulting precipitate was collected. This precipitate was dissolved in a 0.05 M phosphate buffer (pH 8.0) supplemented with 1 M sodium chloride, after which it was dialyzed by the tube method using a 0.05 M phosphate buffer (pH 8.0) supplemented with 1 M sodium chloride as the external fluid, to yield a solution of pertussis toxin (PT), pertussis filamentous hemagglutinin (FHA), pertactin (PRN, 69K-OMP) or pertussis fimbriae (FIM). The concentrate dialyzate was subjected to sucrose gradient density centrifugation on a sucrose density gradient of 1-30 w/w% and at an Rₘₐₓ of 64,900 G for about 18 hours. After completion of the centrifugation, 34 w/w% sucrose was fed into the rotor at a low rate of rotation to collect fractions.

Pertussis toxin (PT) content, pertussis filamentous hemagglutinin (FHA) content, pertactin (PRN, 69K-OMP) content and pertussis fimbriae (FIM) content were determined in the same manner as in Example 1; and endotoxin content, in the same manner as in Reference Example 1. The results are shown in Table 5.

**Table 5**

| Sample | Endotoxin Content (ng/100 µg protein) | Active Ingredient Protein Content (µg protein/ml) | Recovery Rate*(%) |
|---|---|---|---|
| PT | 0.04 | 231.2 | 82.5 |
| FHA | 0.01 | 849.4 | 79.3 |
| FIM | 0.20 | 49.1 | 80.3 |
| 69K-OMP | 0.03 | 319.8 | 92.0 |

| | | | |
|---|---|---|---|
| * Each figure for recovery rate represents a percent ratio relative to the pretreatment value. | | | |

It is evident from this table that endotoxin was selectively removed, with substantially no loss of any protective component, the endotoxin content per 100 µg protein/ml being not more than 1 ng/ml for all components.

### Example 4

To the PT as obtained in Example 3, with addition of amino acid such as Lysine, was added formalin to a final concentration of 0.4 v/v %, and after through mixing, was allowed to stand in an incubator at 39°C for 21-35 days.

To each of FHA, 69K-OMP and FIM as obtained in Example 3, was added formaline to a final concentration of 0.4 v/v %, and after through mixing, was allowed to stand in an incubator at 39°C for 7 days.

Each of these components as treated above was dialyzed against 4 mM phosphate buffer (pH 7.0) supplemented with 0.15M sodium chloride to yield detoxificated PT, inactivated FHA, inactivated 69K-OMP and inactivated FIM.

These detoxificated or inactivated components were blended in several ratios shown in Table 6 and 7, and followed by addition of aluminum chloride to a final concentration of 0.2 mg/ml to give a vaccine respectively.

The results for the experiments of mouse intracerebral potency with these blended vaccines, are shown in Table 6 and 7. The experiments were performed according to the method of Japanese Minimum Requirements for Biological Products (Association of Biologicals Manufactures of Japan).

**Table 6**

| Protein content of respective protective components (µg protein/ml) | | | | Mouse intracerebral potency | |
|---|---|---|---|---|---|
| PT | FHA | FIM | 69K-OMP | IU/ml | 50% effective dose (µg protein) |
| 10 | 40 | 0 | 0 | 10.9 | 1.06 |
| 20 | 30 | 0 | 0 | 13.4 | 0.89 |
| 20 | 40 | 0 | 0 | 11.6 | 1.18 |
| 20 | 50 | 0 | 0 | 13.6 | 1.18 |
| 20 | 80 | 0 | 0 | 12.6 | 1.81 |
| 30 | 40 | 0 | 0 | 19.3 | 0.85 |
| 40 | 40 | 0 | 0 | 18.7 | 1.04 |

**Table 7**

| Potein content of respective protective components (µg protein/ml) | | | | Mouse intravcerebral potency | |
|---|---|---|---|---|---|
| PT | FHA | FIM | 69K-OMP | IU/ml | 50% effective dose (µg protein) |
| 25 | 25 | 0 | 0 | 19.5 | 1.18 |
| 25 | 25 | 5 | 0 | 26.0 | 0.98 |
| 25 | 25 | 0 | 10 | 24.1 | 1.18 |
| 25 | 25 | 5 | 10 | 19.3 | 1.60 |
| 25 | 50 | 0 | 0 | 24.1 | 1.47 |
| 25 | 50 | 5 | 0 | 22.2 | 1.70 |
| 25 | 50 | 0 | 10 | 23.7 | 1.68 |
| 25 | 50 | 5 | 10 | 24.8 | 1.71 |

It is evident from these figures that both inactivated 69K-OMP and inactivated FIM had small effects on the mouse intracerebral potency, and no significant difference were observed among the blended vaccines which contain more than 25µg protein/ml of detoxificated PT.

### Example 5

The experiment of mouse aerozol infection protecting potency were performed with the blended vaccines as obtained in Example 4. Each vaccine diluted to one-third was subcutaneously administered to 4 week-old mouse respectively with 0.2 ml of each diluted one. Four weeks later after the administration, each mouse was subjected to airway infection with 18-323 phase I strain of Bordetella pertussis by using the aerosole chamber, and 10 days later after the infection, the abdomen of each mouse was opened and the trachea and lung were picked out from each infected mice.

The specinen of each homoginized tissue applied to Bordet-Gengou agar. The agar was cultured at 35°C for 5 days and the colonies of Bordetella pertussis were counted.

Based on the colony counts of the non-administered mice, the protective dose was calculated.

The 75% protective dose was calculated in the case of trachea, and the 50% protective dose was calculated in the case of lung. Results were expressed in µg protein.

And growth inhibitory rate was calculated in the high dose administered group. Equation of the growth inhibitory rate was as follows.$\text{Growth inhibitory rate (%)=} \text{(1 -} \frac{\text{Colony counts of High-dose administered mice}}{\text{Colony counts of non-administered mice}} \text{) x 100}$

Results are shown in Table 8.

**Table 8**

| Protein content of respective protective components (µg protein/ml) | | | | Trachea | | Lungs | |
|---|---|---|---|---|---|---|---|
| PT | FHA | FIM | 69K-OMP | 75% Protective dose (µg protein) | Growth inhibitory rate (%) | 50% Protective dose (µg protein) | Growth inhibitory rate (%) |
| 40 | 40 | 0 | 0 | 3.75 | 97.5 | 1.00 | 89.9 |
| 20 | 80 | 0 | 0 | 4.76 | 87.7 | 1.10 | 92.6 |
| 25 | 50 | 0 | 0 | 4.14 | 89.5 | 0.96 | 89.7 |
| 25 | 50 | 5 | 0 | 1.07 | 100 | 0.49 | 100 |
| 25 | 50 | 0 | 10 | 1.74 | 100 | 0.49 | 100 |
| 25 | 50 | 5 | 10 | 0.52 | 100 | 0.26 | 100 |

It is evident from this table that both inactivated 69K-OMP and inactivated FIM ahad small effects on the mouse intracerebral potency, but they showed protective effect on the aerozol infection potency.

### Test Example 1

Differences of adsorption performance between the calcium phosphate gel (In-side Gel) forming and the hydroxyapatite gel (Out-side Gel) on FHA and FIM.

Roux bottle stationary culture prepared by method described in Example 1. The cell culture was concentrated to a one-tenth volume using an ultrafiltration membrane, after which it was centrifuged to separate the supernatant (Sample a) and cells. The cells were dispersed in a one-tenth volume of 0.05 M phosphate buffer (pH 8.0) supplemented with 1 M sodium chloride and stirred well. It was kept standing at 4°C for 4 days, followed by centrifugation to yield the eluted supernatant (Sample b) included PT, FHA, 69K-OMP and FIM.

The culture supernatant (Sample a) and eluted supernatant (Sample b) described above were treated as following 1) or 2).
1) Calcium phosphate Gel (In-side Gel) forming treatment
   To the samples, a 1 M phosphate buffer (pH 8) was added, followed by addition of a calcium acetate solution to a final concentration of 0.5w/v%, 1.0w/v% or 2.0 w/v% and gently stirred at room temperature for 1 hour, followed by centrifugation at 1000 rpm for 10 minutes to supernatant and gel residue. The eluted solution was obtained by eluting the gel residue with a 0.1 M phosphate buffer (pH 8.0) supplemented with a 1M sodium chloride.
2) Hydroxyapatite gel (Out-side gel) treatment
   Hydroxyapatite gel (produced by BDH Chemicals Ltd) was equilibrated with a 0.01M phosphate buffer (pH 8.0). The gel was added to 20w/v%, 10w/v% or 50w/v% to the sample volume and gently stirred at room temperature for 1 hour, followed by centrifugation at 1000 rpm for 10 minutes to seperate supernatant from gel residue. The eluted solution was obtained by eluting the gel residue with a 0.1 M phosphate buffer (pH 8.0) supplemented with a 1M sodium chloride.

The content of FHA or FIM cotent in each sample was determined by ELISA, with FHA or FIM as the house references. The assay results are expressed in µg protein/ml unit. Protein content; Protein precipitated with heated trichloroacetic acid was quantitated by the Lowry method, with bovine serum albumin (Fraction V, produced by Wako Pure Chemical Industries) as a refference. Results are expressed in µg protein/ml unit.

Adsorption rate and recovery rate to the gel on FHA and FIM were calculated by following equations respectively.$\text{Adsorption rate (%)=} \text{(1 -} \frac{\text{Supernatant of post-gel treatment}}{\text{Pre-gel treatment Sample}} \text{) x 100}$$\text{Recovery rate (%)=} \frac{\text{Eluted solution of post-gel treatment}}{\text{Pre-gel treatment Sample}} \text{x 100}$

Results are shown in Table 9 and Table 10

**Table 9**

| | | | | | |
|---|---|---|---|---|---|
| a) Culture supernatant | | | | | |

| | | FHA | | FIN | |
|---|---|---|---|---|---|
| | | Absorption rate (%) | Recovery rate (%) | Absorption rate (%) | Recovery rate (%) |
| Concentration of calcium acetate added (w/v %) | 0.5 | 89.7 | 69.6 | 0.0 | 0.0 |
| | 1.0 | 90.4 | 63.0 | 95.0 | 77.9 |
| | 2.0 | 89.5 | 44.4 | 94.3 | 98.4 |
| Concentration of hydroxyapatite added (w/v %) | 2.0 | 25.4 | 27.5 | 0.0 | 0.0 |
| | 10.0 | 49.5 | 26.4 | 7.9 | 4.1 |
| | 50.0 | 90.8 | 48.9 | 22.1 | 5.3 |

**Table 10**

| | | | | | |
|---|---|---|---|---|---|
| b) The eluted solution from the cell with 0.05M phosphate buffer (pH 8.0) supplemented with 1M-NaCl | | | | | |

| | | FHA | | FIN | |
|---|---|---|---|---|---|
| | | Absorption rate (%) | Recovery rate (%) | Absorption rate (%) | Recovery rate (%) |
| Concentration of calcium acetate added (w/v %) | 0.5 | 96.3 | 87.2 | 18.7 | 12.5 |
| | 1.0 | 98.7 | 61.0 | 99.9 | 74.3 |
| | 2.0 | 98.7 | 55.1 | 99.8 | 94.3 |
| Concentration of hydroxyapatite added (w/v %) | 2.0 | 10.8 | 1.0 | 4.6 | 1.1 |
| | 10.0 | 4.7 | 3.8 | 9.7 | 3.9 |
| | 50.0 | 15.6 | 16.7 | 13.5 | 8.2 |

The calcium phosphate gel (In-side gel) strongly adsorbs both FHA and FIM, but the hydroxyapatite gel has small adsorption effect on the FIM. Also the Hydroxyapatite gel compared with the calcium phosphate gel, has less adsorption effect on the FHA and depend on the volume added.

### Industrial Applicability

The method of the present invention is characterized by the use of the same means of purification for all subject protective components of Bordetella pertussis. Each component can therefore be purified with high efficiency and high recovery rate, an aspect very advantageous for industrial production. It is also possible to efficiently produce an improved purified pertussis component vaccine comprising an effective combination of pertussis filamentous hemagglutinin (FHA), pertactin (PRN, 69K-OMP), pertussis fimbriae (FIM) and pertussis toxin (PT).

## Claims

1. A method of separating at least one member of the group consisting of pertussis filamentous hemagglutinin (FHA), pertactin (PRN, 69K-OMP), pertussis fimbriae (FIM), and pertussis toxin (PT) by bringing a Bordetella Pertussis culture into contact with calcium phosphate gel which is formed by adding calcium ions to the culture in the presence of phosphate ions.

2. A method of separating at least one member of the group consisting of pertussis filamentous hemagglutinin (FHA), pertactin (PRN, 69K-OMP), pertussis fimbriae (FIM) and pertussis toxin (PT) by separating a Bordetella pertussis culture into cells and culture liquid, and carrying out at least one of processes (A), (B), (C) and (D):
(A) a process in which the separated cells are eluted with a salt solution, and pertussis filamentous hemagglutinin (FHA) is separated by bringing the eluted solution into contact with calcium phosphate gel of claim 1,
(B) a process in which the cell residue resulting from the elution treatment of the above process (A) is heated in the presence of a salt solution and brought into contact with calcium phosphate gel, and pertactin (PRN, 69K-OMP) is separated by bringing the eluted solution into contact with calcium phosphate gel of claim 1,
(C) a process in which the cell residue resulting from the elution treatment of the above process (A) is heated in the presence of a salt solution, the supernatant is brought into contact with calcium phosphate gel and eluted with a salt solution, and pertussis fimbriae (FIM) is separated by bringing the eluted solution into contact with calcium phosphate gel of claim 1,
(D) a process in which the culture or the separated culture liquid is brought into contact with calcium phosphate gel of claim 1, and pertussis toxin (PT) is separated from the supernatant.

3. The separation method of claim 2, wherein the supernatant is brought into contact with calcium phosphate gel and eluted with a salt solution to separate pertussis filamentous hemagglutinin (FHA) in process (A).

4. The separation method of claim 2, wherein the supernatant after being brought into contact with calcium phosphate gel is brought into contact with ion exchange gel to separate pertactin (PRN, 69K-OMP) in process (B).

5. The separation method of claim 2, wherein the supernatant is brought into contact with calcium phosphate gel and removed, and the resulting residue is eluted with a salt solution to separate pertussis fimbriae (FIM) in process (C).

6. The separation method of claim 2, wherein the supernatant is brought into contact with ion exchange gel to separate pertussis toxin (PT) in process (D).

7. The separation method of claim 2, wherein the salt solution used in processes (A) and (C) is a buffer containing an alkali metal salt.

8. The separation method of claim 7, wherein the salt solution is a buffer containing 0.01-1.0 M sodium chloride.

9. The separation method of claim 1 or 2, wherein the calcium phosphate gel is formed by adding calcium ions to the culture or the supernatant of pH 7-9 in the presence of phosphate ions.

10. The separation method of claim 9, wherein the equivalent ratio of phosphate ions and calcium ions is 1.25-30 equivalents of phosphate ions per equivalent of calcium ions.

11. The separation method of claim 9, wherein the calcium phosphate gel is formed by adding calcium acetate, as a calcium ion source, at 0.1-2 w/v% in the presence of a 0.05-0.1 M phosphate buffer.

12. The separation method of claim 1 or 2, wherein at least one member of the group consisting of pertussis toxin (PT), pertussis filamentous hemagglutinin (FHA), pertactin (PRN, 69K-OMP) and pertussis fimbriae (FIM) is separated, after which endotoxin is removed by adsorption to aluminum hydroxide gel in the presence of ammonium sulfate.

13. The separation method of claim 1 or 2, wherein at least one member of the group consisting of pertussis toxin (PT), pertussis filamentous hemagglutinin (FHA), pertactin (PRN, 69K-OMP) and pertussis fimbriae (FIM) is separated, after which endotoxin is removed by zonal centrifugation.

14. A pertussis vaccine wherein the components PT:FHA:FIM are admixed in a ratio of 4-6:8-10:1.

15. A pertussis vaccine wherein the components PT:FHA:PRN:FIM are admixed in a ratio of 2-6:4-10:1-2:1.

## Patentansprüche

1. Verfahren zum Abtrennen wenigstens eines Vertreters der Gruppe, die aus filamentösem Pertussis-Hämagglutinin (FHA), Pertactin (PRN, 69K-OMP), Pertussis-Fimbrien (FIM) und Pertussis-Toxin (PT) besteht, indem man eine *Bordetella-pertussis-*Kultur in Kontakt mit Calciumphosphatgel bringt, das durch Zugeben von Calciumionen zu der Kultur in Gegenwart von Phosphationen gebildet wird.

2. Verfahren zum Abtrennen wenigstens eines Vertreters der Gruppe, die aus filamentösem Pertussis-Hämagglutinin (FHA), Pertactin (PRN, 69K-OMP), Pertussis-Fimbrien (FIM) und Pertussis-Toxin (PT) besteht, indem man eine *Bordetella-pertussis-*Kultur in Zellen und Kulturflüssigkeit auftrennt und wenigstens eines der Verfahren (A), (B), (C) und (D) durchführt:
(A) ein Verfahren, bei dem die abgetrennten Zellen mit einer Salzlösung eluiert werden und filamentöses Pertussis-Hämagglutinin (FHA) abgetrennt wird, indem man die eluierte Lösung in Kontakt mit Calciumphosphatgel gemäß Anspruch 1 bringt;
(B) ein Verfahren, bei dem der Zellrückstand, der aus der Elutionsbehandlung des obigen Verfahrens (A) resultiert, in Gegenwart einer Salzlösung erhitzt und in Kontakt mit Calciumphosphatgel gebracht wird und Pertactin (PRN, 69K-OMP) abgetrennt wird, indem man die eluierte Lösung in Kontakt mit Calciumphosphatgel gemäß Anspruch 1 bringt;
(C) ein Verfahren, bei dem der Zellrückstand, der aus der Elutionsbehandlung des obigen Verfahrens (A) resultiert, in Gegenwart einer Salzlösung erhitzt wird, der Überstand in Kontakt mit Calciumphosphatgel gebracht und mit einer Salzlösung eluiert wird und Pertussis-Fimbrien (FIM) abgetrennt werden, indem man die eluierte Lösung in Kontakt mit Calciumphosphatgel gemäß Anspruch 1 bringt;
(D) ein Verfahren, bei dem die Kultur oder die abgetrennte Kulturflüssigkeit in Kontakt mit Calciumphosphatgel gemäß Anspruch 1 gebracht wird und Pertussis-Toxin (PT) aus dem Überstand abgetrennt wird.

3. Trennverfahren gemäß Anspruch 2, wobei in Verfahren (A) der Überstand in Kontakt mit Calciumphosphatgel gebracht und mit einer Salzlösung eluiert wird, so daß filamentöses Pertussis-Hämagglutinin (FHA) abgetrennt wird.

4. Trennverfahren gemäß Anspruch 2, wobei in Verfahren (B) der Überstand,nachdem er in Kontakt mit Calciumphosphatgel gebracht wurde, in Kontakt mit Ionenaustauschergel gebracht wird, so daß Pertactin (PRN, 69K-OMP) abgetrennt wird.

5. Trennverfahren gemäß Anspruch 2, wobei in Verfahren (C) der Überstand in Kontakt mit Calciumphosphatgel gebracht und entfernt wird und der resultierende Rückstand mit einer Salzlösung eluiert wird, so daß Pertussis-Fimbrien (FIM) abgetrennt werden.

6. Trennverfahren gemäß Anspruch 2, wobei in Verfahren (D) der Überstand in Kontakt mit Ionenaustauschergel gebracht wird, so daß Pertussis-Toxin (PT) abgetrennt wird.

7. Trennverfahren gemäß Anspruch 2, wobei die in Verfahren (A) und (C) verwendete Salzlösung ein Puffer ist, der ein Alkalimetallsalz enthält.

8. Trennverfahren gemäß Anspruch 7, wobei die Salzlösung ein Puffer ist, der 0,01-1,0 M Natriumchlorid enthält.

9. Trennverfahren gemäß Anspruch 1 oder 2, wobei das Calciumphosphatgel durch Zugeben von Calciumionen zu der Kultur oder dem Überstand bei pH 7-9 in Gegenwart von Phosphationen gebildet wird.

10. Trennverfahren gemäß Anspruch 9, wobei das Äquivalentverhältnis von Phosphationen zu Calciumionen 1,25-30 Äquivalente Phosphationen pro Äquivalent Calciumionen beträgt.

11. Trennverfahren gemäß Anspruch 9, wobei das Calciumphosphatgel durch Zugeben von Calciumacetat als Calciumionenquelle in einer Menge von 0,1-2% (w/v) in Gegenwart eines 0,05-0,1 M Phosphatpuffers gebildet wird.

12. Trennverfahren gemäß Anspruch 1 oder 2, wobei wenigstens ein Vertreter der Gruppe, die aus Pertussis-Toxin (PT), filamentösem Pertussis-Hämagglutinin (FHA), Pertactin (PRN, 69K-OMP) und Pertussis-Fimbrien (FIM) besteht, abgetrennt wird und anschließend Endotoxin durch Adsorption an Aluminiumhydroxidgel in Gegenwart von Ammoniumsulfat entfernt wird.

13. Trennverfahren gemäß Anspruch 1 oder 2, wobei wenigstens ein Vertreter der Gruppe, die aus Pertussis-Toxin (PT), filamentösem Pertussis-Hämagglutinin (FHA), Pertactin (PRN, 69K-OMP) und Pertussis-Fimbrien (FIM) besteht, abgetrennt wird und anschließend Endotoxin durch Zonenzentrifugation entfernt wird.

14. Pertussis-Impfstoff, wobei die Komponenten PT, FHA und FIM in einem Verhältnis von 4-6:8-10:1 miteinander gemischt sind.

15. Pertussis-Impfstoff, wobei die Komponenten PT, FHA, PRN und FIM in einem Verhältnis von 2-6:4-10:1-2:1 miteinander gemischt sind.

## Revendications

1. Procédé permettant de séparer au moins un élément de l'ensemble que constituent l'hémagglutinine filamentaire coquelucheuse (FHA), la pertactine (PRN, 69K-OMP), la protéine de franges coquelucheuses *(pertussis fimbriae,* FIM) et la toxine coquelucheuse (PT), en mettant une culture de *Bordetella pertussis* en contact avec un gel de phosphate de calcium que l'on forme en ajoutant des ions de calcium à la culture, en présence d'ions phosphate.

2. Procédé permettant de séparer au moins un élément de l'ensemble que constituent l'hémagglutinine filamentaire coquelucheuse (FHA), la pertactine (PRN, 69K-OMP), la protéine de franges coquelucheuses *(pertussis fimbriae,* FIM) et la toxine coquelucheuse (PT), en partageant une culture de *Bordetella pertussis* en cellules et milieu liquide de culture et en mettant en oeuvre au moins l'un des procédés (A), (B), (C) et (D) suivants :
A) un procédé dans lequel on élue les cellules séparées avec une solution de sel et l'on sépare l'hémagglutinine filamentaire coquelucheuse (FHA) en mettant la solution d'élution en contact avec un gel de phosphate de calcium indiqué dans la revendication 1;
B) un procédé dans lequel on chauffe le résidu de cellules résultant de l'opération d'élution du procédé (A) ci-dessus, en présence d'une solution de sel, puis on le met en contact avec un gel de phosphate de calcium, et l'on sépare la pertactine (PRN, 69K-OMP) en mettant la solution d'élution en contact avec un gel de phosphate de calcium indiqué dans la revendication 1 ;
C) un procédé dans lequel on chauffe le résidu de cellules résultant de l'opération d'élution du procédé (A) ci-dessus, en présence d'une solution de sel, puis on met le surnageant en contact avec un gel de phosphate de calcium, on élue celui-ci avec une solution de sel, et l'on sépare la protéine de franges coquelucheuses (FIM) en mettant la solution d'élution en contact avec un gel de phosphate de calcium indiqué dans la revendication 1 ;
D) un procédé dans lequel on met la culture ou le milieu liquide de culture séparé en contact avec un gel de phosphate de calcium indiqué dans la revendication 1, et l'on sépare du surnageant la toxine coquelucheuse (PT).

3. Procédé de séparation conforme à la revendication 2, dans lequel, dans le procédé (A), on met le surnageant en contact avec un gel de phosphate de calcium que l'on élue avec une solution de sel pour séparer l'hémagglutinine filamentaire coquelucheuse (FHA).

4. Procédé de séparation conforme à la revendication 2, dans lequel, dans le procédé (B), après avoir mis le surnageant en contact avec un gel de phosphate de calcium, on le met en contact avec un gel échangeur d'ions pour séparer la pertactine (PRN, 69K-OMP).

5. Procédé de séparation conforme à la revendication 2, dans lequel, dans le procédé (C), on met le surnageant en contact avec un gel de phosphate de calcium, puis on l'élimine et l'on élue le résidu ainsi obtenu avec une solution de sel pour séparer la protéine de franges coquelucheuses (FIM).

6. Procédé de séparation conforme à la revendication 2, dans lequel, dans le procédé (D), on met le surnageant en contact avec un gel échangeur d'ions pour séparer la toxine coquelucheuse (PT).

7. Procédé de séparation conforme à la revendication 2, dans lequel la solution de sel employée dans les procédés (A) et (C) est une solution tampon contenant un sel de métal alcalin.

8. Procédé de séparation conforme à la revendication 7, dans lequel la solution de sel est une solution tampon contenant de 0,01 à 1,0 M de chlorure de sodium.

9. Procédé de séparation conforme à la revendication 1 ou 2, dans lequel on forme le gel de phosphate de calcium en ajoutant des ions de calcium à la culture ou au surnageant, à pH 7-9 et en présence d'ions phosphate.

10. Procédé de séparation conforme à la revendication 9, dans lequel le rapport d'équivalents des ions phosphate et des ions de calcium vaut de 1,25 à 30 équivalents d'ions phosphate par équivalent d'ions de calcium.

11. Procédé de séparation conforme à la revendication 9, dans lequel on forme le gel de phosphate de calcium en ajoutant de l'acétate de calcium, qui sert de source d'ions de calcium, à raison de 0,1 à 2 % p/v, en présence d'une solution tampon de phosphate à 0,05-0,1 M.

12. Procédé de séparation conforme à la revendication 1 ou 2, dans lequel on sépare au moins un élément de l'ensemble que constituent la toxine coquelucheuse (PT), l'hémagglutinine filamentaire coquelucheuse (FHA), la pertactine (PRN, 69K-OMP) et la protéine de franges coquelucheuses (FIM), après quoi l'on élimine l'endotoxine par adsorption sur un gel d'hydroxyde d'aluminium, en présence de sulfate d'ammonium.

13. Procédé de séparation conforme à la revendication 1 ou 2, dans lequel on sépare au moins un élément de l'ensemble que constituent la toxine coquelucheuse (PT), l'hémagglutinine filamentaire coquelucheuse (FHA), la pertactine (PRN, 69K-OMP) et la protéine de franges coquelucheuses (FIM), après quoi l'on élimine l'endotoxine par centrifugation en zones.

14. Vaccin anticoquelucheux dans lequel les composants PT, FHA et FIM se trouvent mélangés en des proportions PT/FHA/FIM de (4-6)/(8-10)/1.

15. Vaccin anticoquelucheux dans lequel les composants PT, FHA, PRN et FIM sont mélangés en des proportions PT/FHA/PRN/FIM de (2-6)/(4-10)/(1-2)/1.
